# EUROPÄISCHE PATENTANMELDUNG

(11) **EP 2 387 939 A1**
(43) Veröffentlichungstag der Anmeldung: **23.11.2011**
(21) Anmeldenummer: 10005315.6
(22) Anmeldetag: 21.05.2010
(51) Int. Cl.: A61B 5/00

(54) **Kleidungsstück und Verfahren zur Erfassung von Entzündungsstellen am menschlichen oder tierischen Körper**

(71) Anmelder: Carl Freudenberg KG, 69469 Weinheim (DE); Universität Heidelberg, 69117 Heidelberg (DE); Klinikum Mannheim GmbH, 68167 Mannheim (DE)
(72) Erfinder: Klatt, Christopher Dr., 69118 Heidelberg (DE); Gretz, Norbert Prof. Dr. med., 68259 Mannheim (DE); Hesser, Juergen, 69118 Heidelberg (DE); Schoenberg, Prof. Dr. Stefan, 68165 Mannheim (DE); Weckbach, Dr. Sabine, 70191 Stuttgart (DE)

(57) **Zusammenfassung**

Ein Kleidungsstück zur Erfassung von Entzündungsstellen am menschlichen oder tierischen Körper, umfassend mindestens eine flächige ebene oder gekrümmte Lage (1), welche derart flexibel ausgestaltet ist, dass sie Bewegungen des Körpers folgt und/oder an diesem anliegt, wobei die Lauge(1) eine Körperseite (2) und eine Aussenseite (3) aufweist, wobei die Körperseite (2) an einen menschlichen oder tierischen Körper anlegbar ist und wobei an der Körperseite (2) Detektoren (4) angeordnet sind, ist im Hinblick auf die Aufgabe, ein Kleidungsstück und ein Verfahren anzugeben, mit welchem ein Entzündungsprofil von Gelenken oder Geweben unter realen Bewegungsbedingungen erfassbar und darstellbar ist, dadurch gekennzeichnet, dass die Detektoren (4) Wärme von Entzündungsstellen und/oder Licht erfassen.

## Beschreibung

### Technisches Gebiet

Die Erfindung betrifft ein Kleidungsstück zur Erfassung von Entzündungsstellen am menschlichen oder tierischen Körper, umfassend mindestens eine flächige ebene oder gekrümmte Lage, welche derart flexibel ausgestaltet ist, dass sie Bewegungen des Körpers folgt und/ oder an diesem anliegt, wobei die Lage eine Körperseite und eine Aussenseite aufweist, wobei die Körperseite an einen menschlichen oder tierischen Körper anlegbar ist und wobei an der Körperseite Detektoren angeordnet sind.

Die Erfindung betrifft auch ein Verfahren zur Erfassung von Entzündungsstellen am menschlichen oder tierischen Körper, wobei eine flächige ebene oder gekrümmte Lage verwendet wird, wobei die Lage den Bewegungen des Körpers folgt und/ oder an diesem anliegt, wobei die Lage eine Körperseite und eine Aussenseite aufweist, wobei die Körperseite an einen menschlichen oder tierischen Körper angelegt wird und wobei an der Körperseite Detektoren angeordnet werden.

### Stand der Technik

Aus dem Stand der Technik sind Kleidungsstücke der eingangs genannten Art bereits bekannt.

So ist aus der DE 10 2004 030 261 A1 ein Kleidungsstück, nämlich ein Body, bekannt, welcher Dehnungsmesstreifen und Sensoren zum Messen des Hautwiderstands oder von Körpertemperaturen aufweist. Diese Sensoren müssen auch während der Bewegung ständig in Körperkontakt gehalten werden. Hierzu wird vorgeschlagen, den Body mit Elastanfäden auszustatten. Aus dieser Druckschrift ist auch bekannt, elastomere Sensoren einzusetzen, wobei das Elastomer mit leitfähigen Partikeln befüllt ist.

Vor diesem Hintergrund ist aus der DE 10 2004 015 682 B4 bekannt geworden, in den Körper eines Lebewesens den Stoff Indozyaningrün (ICG) zu injizieren. Dieser Farbstoff wird durch Einstrahlung optischer Strahlung zu fluoreszierender Strahlung angeregt. Die fluoreszierende Strahlung kann erfasst werden, um beispielsweise Aussagen über die Dicke von Gewebeschichten zu machen.

Entzündliche Gelenkerkrankungen, wie die rheumatoide Arthritis, Psoriasisarthritis oder Gichtarthritis sind chronisch-progressive entzündliche Skeletterkrankungen unklarer Ätiologie. Diese Erkrankungen verursachen chronische Schmerzen, führen zu einer progressiven Gelenkdestruktion und dadurch bedingten Invalidität. Sie haben durch die erhöhte Morbidität, Mortalität, Einschränkungen der Lebensqualität und Erwerbsfähigkeit erhebliche Auswirkungen nicht nur auf das Individuum, sondern auf die gesamte Gesellschaft.

Derzeit sind etwa 0,5 - 2 % der Menschheit von rheumatoider Arthritis betroffen. Dies bedeutet, dass allein in Deutschland mehr als 400000 Menschen von dieser Krankheit betroffen sind. Die mit der Behandlung dieser Krankheit einhergehenden Kosten sind aufgrund deren chronischen Verlaufs mit intermittierenden aktiven Phasen außerordentlich hoch. Es besteht daher ein Bedarf, dieses Krankheitsbild nicht nur möglichst früh zu erkennen, sondern auch dessen Fortschreitung an der zu behandelnden Person exakt zu erfassen und zu vermessen.

Insbesondere sollen entzündliche Erkrankungen früh detektiert und ein therapeutisches Ansprechen, beispielsweise auf TNF-Alpha-Blocker, dokumentiert und quantitativ erfasst werden können, noch bevor strukturelle Schäden entstehen. Diese strukturellen Schäden sind meist erst relativ spät im Röntgenbild erkennbar.

Die aus dem Stand der Technik bekannten Vorrichtungen und Verfahren sind allerdings nur wenig geeignet, Entzündungsstellen am Körper eines Menschen oder eines Tieres während dessen natürlicher Bewegung - quasi im Alltag und vor Ort- zu erfassen. Vielmehr existieren bisher lediglich stationäre Apparate, an denen ein Körper in einem relativ statischen Zustand nur in einer Klinik oder in einer Arztpraxis untersucht werden kann.

### Darstellung der Erfindung

Der Erfindung liegt daher die Aufgabe zugrunde, ein Kleidungsstück und ein Verfahren anzugeben, mit welchem ein Entzündungsprofil von Gelenken oder Geweben unter realen Bewegungsbedingungen erfassbar und darstellbar ist.

Die vorliegende Erfindung löst die zuvor genannte Aufgabe durch die Merkmale des Patentanspruchs 1.

Danach erfassen die Detektoren Wärme von Entzündungsstellen und/ oder Licht.

Erfindungsgemäß wird das hier beschriebene Kleidungsstück verwendet, um Entzündungsstellen des menschlichen oder tierischen Körpers zu erfassen und optisch darzustellen. Insbesondere können hierdurch Rheumaherde und Infektionen erkannt und dargestellt werden. Das Kleidungsstück kann insbesondere zur Erfassung von Entzündungsstellen bei Diabetikern im Fußbereich verwendet werden. Das hier beschriebene Kleidungsstück passt sich erfindungsgemäß an die Form des menschlichen oder tierischen Köpers an und folgt dessen Bewegung. Beim Folgen der Bewegung liegt das Kleidungsstück am Körper derart an, dass die Detektoren in jedem Krümmungsszustand eines Gelenks die Entzündungsstellen an diesem erfassen können. Insoweit ist eine krümmungs- und bewegungsabhängige Entzündungsmustererkennung realisierbar. Insoweit ist ein Entzündungsprofil von Gelenken oder Geweben unter realen Bewegungsbedingungen erfassbar und darstellbar.

Folglich ist die eingangs genannte Aufgabe gelöst.

Es könnten Lichtquellen vorgesehen sein, welche anregendes Licht abstrahlen, wobei die Detektoren emittiertes Licht erfassen. Hierbei ist erkannt worden, dass Entzündungsstellen Leckagestellen des menschlichen oder tierischen Körpers darstellen, an denen Proteine austreten. Sofern an die Proteine Marker angebunden sind, können diese durch anregendes Licht zur Fluoreszenz angeregt werden. Das von den Markern emittierte Licht kann dann von den Detektoren erfasst werden. Das emittierte Licht erlaubt die Identifizierung einer Entzündungsstelle.

Eine Lichtquelle und ein Detektor könnten in einem Bauteil integriert sein, Hierdurch ist ein apparativ einfacher Aufbau des Kleidungsstücks möglich. Die Detektoren können die erfassten Daten entweder vor Ort - quasi in einem integrierten Prozessor - verarbeiten oder die Daten zu einer vom Kleidungsstück entfernten Hardware übertragen. Dabei ist denkbar, dass die Übertragung per Funk, per Infrarotstrahlung oder per Ultraschall erfolgt.

Die Detektoren könnten LEDs und/ oder Photodioden umfassen. Diese Detektoren sind kommerziell leicht verfügbar und technisch ausgereift. Hierbei ist konkret denkbar, dass LEDs oder Photodioden auf organischer Basis verwendet werden.

Durch Temperaturdetektoren kann die Körpertemperatur an Entzündungsstellen erfasst werden. Diese Detektoren könnten als Dioden, Transistoren oder als Sensoren auf organischer Basis ausgestaltet sein. Insbesondere könnten Feldeffekttransistoren (FETs) verwendet werden.

Die Lichtquelle könnte mindestens eine lichtleitende Polymerfaser aufweisen, welche anregendes Licht einstrahlt. Durch diese konkrete Ausgestaltung können Polymerfaserbündel verwendet werden, welche das anregende Licht in einem Punktmuster einstrahlen.

Vor diesem Hintergrund könnte der Detektor mindestens eine lichtleitende. Polymerfaser aufweisen, welche emittiertes Licht erfasst. Je nach Anzahl der verwendeten Polymerfasern kann so die Auflösung eingestellt werden, mit welcher eine Entzündungsstelle untersucht wird. Die Polymerfaserbündel könnten mit einem Kamerachip verbunden sein. Hierbei ist konkret denkbar, 256 x 256 Fasern einzusetzen. Die Polymerfasern können das emittierte Licht zu einer Hardware transprotieren, die von der Entzündungsstelle beabstandet ist.

Die Detektoren könnten derart angeordnet sein, dass sie ein regelmäßiges oder unregelmäßiges Raster bilden, wobei an jeweils einem Rasterpunkt ein Detektor angeordnet ist. Hierdurch ist eine flächige Darstellung des Entzündungsprofils realisierbar. Vorzugsweise sind die Detektoren an Gelenken des menschlichen oder tierischen Körpers angeordnet. Die Defektoren können die erfassten Daten entweder vor Ort - quasi in einem integrierten Prozessor - verarbeiten oder die Daten zu einer vom Kleidungsstück entfernten Hardware übertragen. Dabei ist denkbar, dass die Übertragung per Funk, per Infrarotstrahlung oder per Ultraschall erfolgt.

Die Lage könnte Krümmungsmesser aufweisen. Hierdurch kann der Bewegungszustand oder Krümmungszustand eines Körperteils oder Gelenks des Körpers erfasst werden. Eine Entzündungsstelle kann in jedem Bewegungszustand des menschlichen Körpers untersucht werden. Die Krümmungsmesser können bei Dehnung ihre elektrischen Widerstandswerte ändern. Hierdurch kann ein spezifischer Krümmungszustand eines Körperteils, beispielsweise eines Fingers, Beins oder Gelenks, ermittelt werden.

Das Kleidungsstück könnte als Handschuh ausgestaltet sein. Hierdurch können die Fingergelenke im Hinblick auf Entzündungsstellen untersucht werden.

Die eingangs genannte Aufgabe wird auch durch ein eingangs genanntes Verfahren zur Erfassung von Entzündungssteten am menschlichen oder tierischen Körper gelöst.

Bei diesem Verfahren werden die Detektoren verwendet, um Entzi7ndungsstellen durch Wärme und/ oder Licht zu erfassen.

Mit diesem Verfahren kann eine Diagnose oder Verlaufskontrolle von Rheuma, Entzündungen oder Läsionen durchgeführt werden. Um Wiederholungen in Bezug auf die erfinderische Tätigkeit zu vermeiden, sei auf die Ausführungen zum Kleidungsstück als solchem verwiesen.

Zur Durchführung des Verfahrens wird bevorzugt das zuvor beschriebene Kleidungsstück verwendet

Ein Marker könnte im Blutkreislauf des Körpers durch Lichtquellen angeregt werden, emittiertes Licht abzustrahlen, wobei das emittierte Licht durch Detektoren an Gelenken und/ oder auf der menschlichen Haut erfasst wird. Um Wiederholungen in Bezug auf die erfinderische Tätigkeit zu vermeiden, sei auf die Ausführungen zum Kleidungsstück als solchem verwiesen.

Als Marker könnte Indozyaningrün (ICG) verwendet werden. Indozyaningrün (ICG) erlaubt heute eine hohe räumliche und zeitliche Auflösung der chorioidalen und retinalen Gefäße und seiner pathologischen Veränderungen. ICG weist im Vergleich zu Fluorescein zwei charakteristische Vorteile auf. Leckagen werden reduziert, da es ein hohes Eiweißbindungsvermögen zeigt. Des Weiteren erfährt es eine relativ geringe Fluoreszenzblockade durch Blut und Pigmente. Dieser Marker wird vorteilhaft an Eiweiß gebunden. Zwar entfernt die Leber problemlos den Marker von Proteinen, jedoch können mit Marker versehene Proteine an Entzündungsstellen des menschlichen Körpers austreten und daher im Sinne einer Leckage erkannt und erfasst werden.

Der Marker könnte durch Licht einer Wellenlänge von 700- 860 nm, bevorzugt von 760 nm, zur Fluoreszenz angeregt werden und Licht einer Wellenlänge von 820 nm emittieren. Der Wellenlängenbereich 700 - 860 nm hat sich als besonders voteilhaft im Hinblick auf seine Eindringtiefe in menschliches Gewebe erwiesen.

Das hier beschriebene Kleidungsstück sowie das Verfahren dienen der Detektion, dem Staging, der Verlaufskontrolle und der Rezidiverkennung von rheumatischen Erkrankungen, die sich flächenartig ausprägen. Die Detektion erfolgt unter Verwendung geeigneter fluoreszierender Diagnostika, die im Interstitium nachweisbar sind. Des Weiteren können Entzündungen durch Temperaturmessung erkannt werden. Hierzu zählen auch lokalisierte oder generalisierte Infektionen. Es kann eine Rekonstruktion der Entzündungsstelle, insbesondere nach offenen Operationen, erfolgen. Des Weiteren können Tumorerkrankungen erkannt werden, die sich durch Messungen an der Hautoberfläche durch Signale wie Hautwiderstandsänderungen detektieren lassen.

Es gibt nun verschiedene Möglichkeiten, die Lehre der vorliegenden Erfindung in vorteilhafter Weise auszugestalten und weiter zu bilden. Dazu ist einerseits auf die nachgeordneten Ansprüche, andererseits auf die nachfolgende Erläuterung bevorzugter Ausführungsbeispiele des erfindungsgemäßen Kleidungsstücks anhand der Ausführungsbeispiele zu verweisen.

In Verbindung mit der Erläuterung der bevorzugten Ausführungsbeispiele werden auch im Allgemeinen bevorzugte Ausgestaltungen und Weiterbildungen der Lehre erläutert.

### Kurzbeschreibung der Zeichnung

In der Zeichnung zeigen
- Fig. 1: ein Kleidungsstück, welches als Handschuh ausgestaltet ist, auf dem Detektoren angeordnet sind, und
- Fig. 2: eine Schnittansicht eines Fingers eines Handschuhs, dessen Körperseite mit Detektoren ausgerüstet ist.

### Ausführung der Erfindung

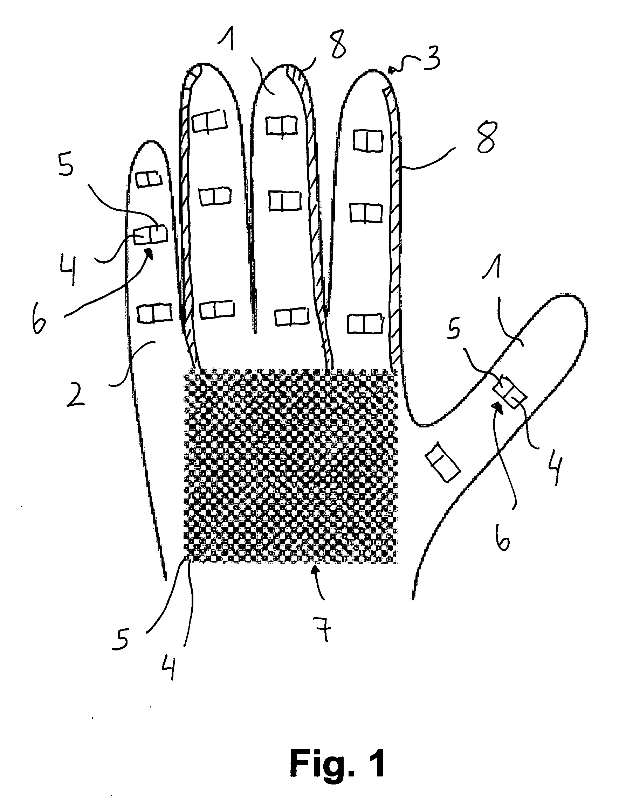

Fig. 1 zeigt ein Kleidungsstück zur Erfassung von Entzündungsstellen am menschlichen oder tierischen Körper, umfassend mindestens eine flächige ebene oder gekrümmte Lage 1, welche derart flexibel ausgestaltet ist, dass sie' Bewegungen des Körpers folgt und/ oder an diesem anliegt, wobei die Lage1 eine Körperseite 2 und eine Aussenseite 3 aufweist, wobei die Körperseite 2 an einen menschlichen oder tierischen Körper anlegbar ist und wobei an der Körperseite 2 Detektoren 4 angeordnet sind.

Es sind Detektoren 4 vorgesehen, welche Wärme und/ oder Licht detektieren können. Die Detektoren 4 erfassen Wärme von Entzündungsstellen und/ oder Licht. Das Licht wird von den Entzündungsstellen emittiert.

Es sind Lichtquellen 5 vorgesehen, welche anregendes Licht abstrahlen, wobei die Detektoren 4 emittierte Licht erfassen. Eine Lichtquelle 5 und ein Detektor 4 sind in einem Bauteil 6 integriert.

Die Detektoren 4 sind derart angeordnet, dass sie ein regelmäßiges Raster 7 bilden, wobei an jeweils einem Rasterpunkt ein Detektor 4 angeordnet ist.

Die Lage 1 weist Krümmungsmesser 8 auf. Diese können die Krümmung eines Körperteils erfassen. Dies kann durch Spannungsmessung erfolgen. Denkbar ist auch, oberhalb und unterhalb eines Fingers eine Faser zu führen. Dann kann die Krümmung eines Fingers durch Verschiebung der Faser gemessen werden. Diese Verschiebung kann optisch oder elektronisch gemessen werden.

Das Kleidungsstück ist als Handschuh ausgestaltet. Die Lage 1 ist aus Latex oder Jersey gefertigt und flexibel dehnbar und drapierbar.

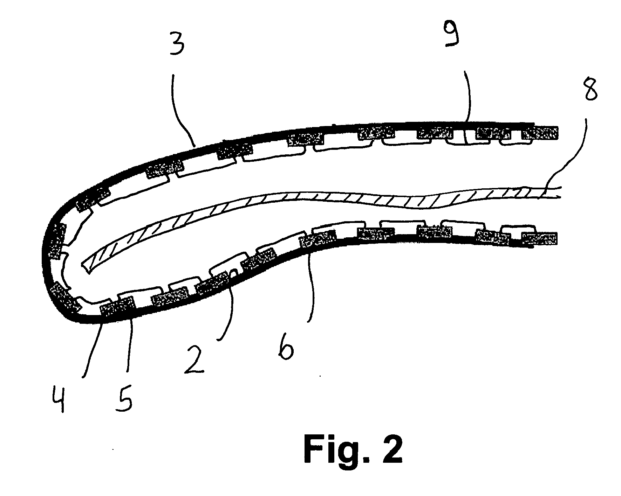

Fig. 2 zeigt einen Finger eines Handschuhs zur Erfassung von Entzündungsstellen am menschlichen oder tierischen Körper, umfassend mindestens eine flächige ebene oder gekrümmte Lage 1, welche derart flexibel ausgestaltet ist, dass sie Bewegungen des Körpers folgt und/oder an diesem anliegt, wobei die Lage 1 eine Körperseite 2 und eine Aussenseite 3 aufweist, wobei die Körperseite 2 an einen menschlichen oder tierischen Korper anlegbar ist und wobei an der Körperseite 2 Detektoren 4 angeordnet sind.

Es sind Detektoren 4 vorgesehen, welche Wärme und/ oder Licht detektieren können. Die Detektoren 4 erfassen Wärme von Entzündungsstellen und/ oder Licht. Das Licht wird von den Entzündungsstellen emittiert.

Es sind Lichtquellen 5 vorgesehen, welche anregendes Licht abstrahlen, wobei die Detektoren 4 emittiertes Licht erfassen. Eine Lichtquelle 5 und ein Detektor 4 sind in einem Bauteil 6 integriert.

Die Detektoren 4 sind untereinander mit Leitungen 9 verbunden, weiche die Detektoren 4 mit Strom versorgen und/oder einen Datentransfer zu einer entfernten Hardware erlauben. Die Signale der Detektoren 4 auf der Körperseite 2 werden verstärkt und digitalisiert. Die Hardware kann die Daten über das Internet zu einem Zentralcomputer oder einer zentralen Datenbank versenden. Dort kann eine umfassende Diagnose durchgeführt und eine Therapie erarbeitet werden.

Die Lage 1 weist Krümmungsmesser 8 auf. Diese können die Krümmung eines Körperteils erfassen. Konkret können hier Winkel eines gekrümmten Fingers gemessen werden.

Mit den zuvor beschriebenen Kleidungsstücken ist ein Verfahren zur Erfassung von Entzündungsstellen am menschlichen oder tierischen Körper durchführbar, wobei eine flächige ebene oder gekrümmte Lage 1 verwendet wird, wobei die Lage 1 den Bewegungen des Körpers folgt und/ oder an diesem anliegt, wobei die Lage 1 eine Körperseite 2 und eine Aussenseite 3 aufweist wobei die körperseite 2 an einen menschlichen oder tierischen Körper angelegt wird und wobei an der Körperseite 2 Detektoren 4 angeordnet werden.

Die Detektoren 4 werden verwendet, um Entzündungsstellen durch deren Wärme und/ oder durch Licht zu erfassen, welches von den Entzündungsstellen emittiert wird.

Ein Marker im Blutkreislauf des Körpers wird durch Lichtquellen 5 angeregt, emittiertes Licht abzustrahlen, wobei das emittierte Licht durch Detektoren 4 an Gelenken und/ oder auf der menschlichen Haut erfasst wird.

Als Marker wird Indozyaningrün (ICG) verwendet. Der Marker wird durch Licht einer Wellenlänge von 760 nm zur Fluoreszenz angeregt und emittiert Licht einer Wellenlänge von 820 nm.

Hinsichtlich weiterer vorteilhafter Ausgestaltungen und Weiterbildungen der erfindungsgemäßen Lehre wird einerseits auf den allgemeinen Teil der Beschreibung und andererseits auf die beigefügten Patentansprüche verwiesen.

Abschließend sei ganz besonders hervorgehoben, dass die zuvor ausgewählten Ausführungsbeispiele lediglich zur Erörterung der erfindungsgemäßen Lehre dienen, diese jedoch nicht auf diese Ausführungsbeispiele einschränken.

## Patentansprüche

1. Kleidungsstück zur Erfassung von Entzündungsstellen am menschlichen oder tierischen Körper, umfassend mindestens eine flächige ebene oder gekrümmte Lage (1), welche derart flexibel ausgestaltet ist, dass sie Bewegungen des Körpers folgt und/ oder an diesem anliegt, wobei die Lage (1) eine Körperseite (2) und eine Aussenseite (3) aufweist, wobei die Körperseite (2) an einen menschlichen oder tierischen Körper anlegbar ist und wobei an der Körperseite (2) Detektoren (4) angeordnet sind,
**dadurch gekennzeichnet, dass** die Detektoren (4) Wärme von Entzündungsstellen und/ oder Licht erfassen.

2. Kleidungsstück nach Anspruch 1, **dadurch gekennzeichnet, dass** Lichtquellen (5) vorgesehen sind, welche anregendes Licht abstrahlen, wöbei die Detektoren (4) emittiertes Licht erfassen.

3. Kleidungsstück nach Anspruch 1 oder 2, **dadurch gekennzeichnet, dass** eine Lichtquelle (5) und ein Detektor (4) in einem Bauteil (6) integriert sind.

4. Kleidungsstück nach einem der Ansprüche 1 bis 3, **dadurch gekennzeichnet, dass** die Lichtquelle (5) mindestens eine Polymerfaser aufweist, welche anregendes Licht einstrahlt.

5. Kleidungsstück nach einem der Ansprüche 1 bis 4, **dadurch gekennzeichnet, dass** der Detektor (4) mindestens eine Polymerfaser aufweist, welche emittiertes Licht erfasst.

6. Kleidungsstück nach einem der Ansprüche 1 bis 5, **dadurch gekennzeichnet, dass** die Detektoren (4) derart angeordnet sind, dass sie ein regelmäßiges oder unregelmäßiges Raster (7) bilden, wobei an jeweils einem Rasterpunkt ein Detektor (4) angeordnet ist.

7. Kleidungsstück nach einem der Ansprüche 1 bis 6, **dadurch gekennzeichnet, dass** die Lage (1) Krümmungsmesser (8) aufweist

8. Verfahren zur Erfassung von Entzündungsstellen am menschlichen oder tierischen Körper, wobei eine flächige ebene oder gekrümmte Lage (1) verwendet wird, wobei die Lage (1) den Bewegungen des Körpers folgt und/ oder an diesem anliegt, wobei die Lage (1) eine Körperseite (2) und eine Aussenseite (3) aufweist, wobei die Körperseite (2) an einen menschlichen oder tierischen Körper angelegt wird und wobei an der Körperseite (2) Detektoren (4) angeordnet werden,
**dadurch gekennzeichnet, dass** die Detektoren (4) verwendet werden, um Entzündungsstellen durch deren Wärme und/ oder durch Licht zu erfassen, welches von den Entzündungsstellen emittiert wird.

9. Verfahren nach Anspruch 8, **dadurch gekennzeichnet, dass** ein Marker im Blutkreislauf des Körpers durch Lichtquellen (5) angeregt wird, emittiertes Licht abzustrahlen, wobei das emittierte Licht durch Detektoren (4) an Gelenken und/ oder auf der menschlichen Haut erfasst wird.

10. Verfahren nach Anspruch 9, **dadurch gekennzeichnet, dass** als Marker Indozyaningrün (ICG) verwendet wird.

11. Verfahren nach Anspruch 9 oder 10, **dadurch gekennzeichnet, dass** der Marker durch Licht einer Wellenlänge von 760 nm zur Fluoreszenz angeregt wird und Licht einer Wellenlänge von 820 nm emittiert.
